(19) **Europäisches Patentamt / European Patent Office / Office européen des brevets**

(11) **EP 3 625 437 B1**

(12) **EUROPEAN PATENT SPECIFICATION**

(45) Date of publication and mention of the grant of the patent:
**26.07.2023 Bulletin 2023/30**

(21) Application number: **18730111.4**

(22) Date of filing: **15.05.2018**

(51) International Patent Classification (IPC):
***E21D 11/08*** (2006.01)

(52) Cooperative Patent Classification (CPC):
**E21D 11/086; E21D 11/08**

(86) International application number:
**PCT/IB2018/053379**

(87) International publication number:
**WO 2018/211414 (22.11.2018 Gazette 2018/47)**

(54) **PRE-CAST SEGMENT FOR TUNNELS AND METHOD FOR PRODUCING AND MONITORING SAID PRE-CAST SEGMENT**

VORGEGOSSENES SEGMENT FÜR TUNNEL UND VERFAHREN ZUR HERSTELLUNG UND ÜBERWACHUNG DES VORGEGOSSENEN SEGMENTS

SEGMENT PRÉ-COULÉ POUR TUNNELS ET PROCÉDÉ DE PRODUCTION ET DE SURVEILLANCE DUDIT SEGMENT PRÉ-COULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2017 IT 201700052365**

(43) Date of publication of application:
**25.03.2020 Bulletin 2020/13**

(73) Proprietor: **Safecertifiedstructure Tecnologia S.p.A.**
**00198 Roma (IT)**

(72) Inventor: **MANCINI, Giuseppe**
**10137 Torino (IT)**

(74) Representative: **Locas, Davide et al**
**Cantaluppi & Partners S.r.l.**
**Piazzetta Cappellato Pedrocchi, 18**
**35122 Padova (IT)**

(56) References cited:
**FR-A1- 3 033 586     JP-A- 2005 273 445**
**JP-A- 2011 058 199     JP-B2- 3 048 027**

**Description**

Technical field

The present invention relates to a pre-cast segment for tunnels and to an associated production and monitoring method having the features detailed in the preamble of the respective independent claims.

Technical background

Tunnels are currently formed by digging a tunnelway typically of circular cross section by means of a large and costly drilling machine known as a TBM or tunnel boring machine, and, as work proceeds, by progressively lining the walls of the tunnelway dug in this way with pre-cast segments made of reinforced concrete. Since, once they have been placed in position, the segments, which in practice form sectors of a cylindrical ring that are moved towards one another so as to form a complete ring and, ring after ring, cover the entire tunnel, cannot be replaced in the event of damage, it is particularly important to check their integrity from the first step of construction and then at steps of the installation process, because if their integrity is compromised this may result in significant structural damage to the tunnel as a whole.

This requires monitoring of the tensions, stresses and deformations present within the structure.

As is known, besides representing the fundamental cement structures of tunnels, the segments also represent the most critical structural part of said tunnels.

It may often occur that pre-cast segments become structurally compromised due to various factors, some of which are cited briefly below:

- problems occurring during the production process (whether in the step of concrete casting or in the step of curing) that have introduced structural defects, possibly positioned in the deep layers of the segments and thus impossible to detect by means of a visual inspection,
- unexpected impacts or stresses applied during the steps of transport from the production site to the installation site,
- impacts or stresses applied during the installation of the segments in a tunnel,
- impacts or stresses applied during processes following the installation of the segments,
- impacts or stresses to which the segments are subjected as a result of natural external factors (landslides, earthquakes, etc.) or artificial external factors (production of boreholes for other tunnels in the vicinity, production of foundations for building structures close to the tunnel, etc.).

In this context, the term structural defects is understood to mean all types of defects that can occur within a structure (for example defects at intermittent points, linear defects, planar defects, composition-related defects, etc.).

It is clear that, in the case of the presence of one or more of the aforementioned factors, fractures and cracks in the cement structures can develop, also leading to serious consequences from a structural viewpoint, and thus posing a risk of severely compromising the availability and safety of the tunnel itself.

Currently, such possible structural fractures or cracks of the segments of a tunnel are difficult to detect, primarily because segment samples are studied and tested with regard to their resistance to mechanical forces (load tests, etc.) and high temperatures (fire resistance tests, etc.) in specialised laboratories, however few tests have been developed for monitoring the behaviour of said segments continuously from the production step to the step of disposal, with particular reference to the behaviour of the segment during its actual use in a tunnel.

In fact, checks are typically performed inside the tunnels in accordance with a scheduled plan (and therefore independently of potential hazard situations in the tunnel) or following indications of detectable surface damage (and which damage is thus intrinsically liable to manifest itself belatedly).

Hence, the checks performed, once a need therefor has been established, are therefore often based on optical and/or acoustic interactions (for example checks using optical fibres or ultrasound sensors) in order to assess, in principle and in a limited manner, any macro movements of large sections of tunnel, suspected of having been subjected to severe and harmful cracks .

It is also important to note that such study phases can entail a reduction of the availability of the tunnel, if not even the complete lack of usability thereof for vehicles travelling, due to the presence of the equipment used to study the integrity of the structure.

It is clear that such analyses have various disadvantages detailed further below. Firstly, when proceeding with an analysis of the segment in the presence of a potential structural hazard or risk situation, there is the inherent problem of defining the risk level from which an analysis of the integrity of the structure should be activated. This means that it is possible that some phenomena might be capable of inducing fracturing, deformations and cracks (possibly in portions that cannot be easily analysed from the inner side of the tunnel), as a result of which the segments are structurally compromised without there having been a natural or artificial phenomenon of such intensity as to have alerted the bodies responsible for assessing the possible risk of subsidence of the structure. In this case, any intervention for repairing the resultant damage will be performed precariously late. Another disadvantage linked to the techniques typically used in this technical field is that, following an indication of potential subsidence or a previously scheduled analysis of the structures of the tunnel, a worker has to physically perform the analysis of the structure, which results in a significant expenditure of time in order to reach the place indicated and to actually carry out the analysis.

It is clear that such an analysis method does not allow a great timeliness of the response when there is a suspicion of, or information indicating a possible structural subsidence, and even less so allows an interpretation of structural conditions that can develop into situations dangerous for the stability of the tunnel.

It is also important to note that the measurement of a tension within a solid body presents significant difficulties and is usually obtained indirectly by measuring deformations on the outer surface of the elements constituting the structure or directly within the same. The transition from measuring a deformation to measuring a tension is possible on the basis of the prior knowledge of the basic law of the material being measured. This transition is simple and reliable for linear resilient materials, the mechanical properties of which are as follows: constant over time and uniform in space within the structure itself, known accurately and having low statistical scattering. This category of materials for example includes metals such as steels and aluminium alloys.

Greater difficulties arise when it is sought to measure a tension within a structure, the material characteristics of which are neither uniform in space nor constant over time and are not generally known accurately beforehand, as is the case for example for all cement agglomerates. Further difficulties are encountered on the basis of the viscoelastic nature of the cement material (a characteristic that can be correlated to agglomerates in general), which tends to manifest itself in the form of deformations and non-constant states of tension, both in the short term and in the long term, and also on the basis of continuously applied loads. It is therefore clear that analyses performed only at the surface on one or more macro portions of tunnel pose the specific risk of not being able to be correctly correlated to the deformations actually present in the segments, of not being representative of an actual state of compromise of the segments, and of not allowing timeliness or efficacy of the action/response.

A further know solution is disclosed in FR3033586, relating to a wall element instrumented with at least one sensor and an apparatus comprising at least one reading unit connected to at least said sensor.

Furthermore JP3048027B discloses a movable form device for tunnels, allowing to perform form positioning work easily in a short time and position a form correctly into a specified position.

JP2011058199 relates to use of a plurality of segments arranged in a row in the circumferential direction of a tunnel to form a tunnel wall. The segments are provided with both a measuring means for measuring physical quantities for detecting contact between a specific location of the outer surface of the segments facing the ground side and a flowing material filled in a gap between the ground and the segments when the segments have been arranged in the tunnel and a communication means for transmitting information corresponding to measurement results of the measuring means to the other device by wireless communication.

Description of the invention

The object of the present invention is to provide a pre-cast segment for tunnels and an associated production and manufacturing method that overcome the disadvantages of the identified prior art.

Within the scope of this object, the objective of the invention is to provide a product and a method that make it possible to determine and continuously and effectively monitor the structural integrity of pre-cast segments of tunnel from the time of production thereof to the time of full utilisation thereof.

The inventive product produced in accordance with the present invention is a pre-cast segment for a reinforced concrete tunnel, comprising an arcuate structure having a reinforcement and a cement agglomerate that is designed to cover structurally repeated annular tunnel segments for modules corresponding to a fraction of the cross section thereof.

The arcuate structure of the segment preferably comprises respective opposite radial faces that lie on planes that are angularly spaced apart from one another and passing along a longitudinal axis of the tunnel, respective opposite circumferential faces that lie on surfaces perpendicular to said longitudinal axis and are spaced apart along said longitudinal axis, and respective opposite longitudinal faces that lie on surfaces that are parallel to said longitudinal axis and spaced apart from one another radially with respect to said longitudinal axis, said radial faces being adapted to be moved towards respective radial faces of adjacent segments in order to form an annular tunnel portion, said circumferential faces being adapted to be moved towards respective circumferential faces of adjacent segments in order to form a linear extent of said tunnel along said longitudinal axis and an outer longitudinal face being at a greater distance than an inner longitudinal face from said longitudinal axis, is placed in contact with the ground of said tunnel, wherein at least one investigation device is embedded in said arcuate structure of said pre-cast segment at a predetermined distance from at least one of said radial, circumferential or longitudinal faces, so as to detect predetermined structural parameters.

In this context, the term agglomerate is understood to mean a material in which there is a binder (for example cement, bitumen, lime, polymer resin, etc.) and a bonded material (for example sand, gravel, clay, siliceous powders, glass fibres, steel fibres, carbon fibres, aramid fibres, etc.).

It is important to note that, thanks to the features of the present invention, it is possible to determine and monitor the state of integrity of the aforementioned segment also continuously, by checking and monitoring the desired characteristics in real time.

The investigation device advantageously can be an accelerometer, an extensometer, an inclinometer, a capacitive sensor, a heat sensor, an optical sensor, an acoustic sensor, or another type of sensor known in the art. Accordingly, the predetermined structural parameters are therefore, for example, the tension detected at a given point, the deformation detected at a specific point, the orientation, the temperature, the variation in mechanical-structural characteristics, etc.

In accordance with one embodiment, the investigation device comprises a deformable body in which at least one deformation meter is arranged, which is configured to detect at least three deformation measures oriented with respect to one another, such that a tension within said investigation device is proportional to a combination of said three deformation measures.

The deformable body preferably has purely resilient behaviour, devoid of viscous or viscoelastic characteristics, at least with regard to the stresses permissible in the structure of a cement agglomerate. In this way, it is possible to detect a tension correlated to deformations occurring within the device (and therefore within the segment) rather than outside the device (segment). Such a characteristic signifies a significant technical advantage, since, because the deformable body is disposed within the investigation device, in an undisturbed zone, the deformation meter detects the deformations of the device, this being a resilient solid, providing a measure correlated only to the applied external actions and not influenced by viscous phenomena, as would result if the aforementioned deformable body with resilient behaviour were omitted.

In this context, the term combination means a mathematical combination of the aforementioned values. In a particular case, when the values in question relate to linear vector spaces, the aforementioned combination can be represented by means of a linear combination of the three deformation measures relating to linear vector spaces.

In addition, in this context, an action is defined as any cause or set of causes capable of inducing states of stress and/or deformation in a structure. The actions can be classified as follows in accordance with the way in which they are performed:

- direct actions, induced by concentrated forces, fixed or mobile distributed loads;
- indirect actions, induced by impressed movements, changes in temperature and humidity, shrinkage, precompression, constraint subsidence, effects of viscosity. External actions mean both explicit actions (or direct actions) and implicit actions (or indirect actions).

In a preferred embodiment the deformable body is produced using materials having a modulus of elasticity equal to, or strictly greater than, that of the arcuate structure produced from cement agglomerate in which said deformable body is embedded, so as to reduce and even out the irregularity of contact between the cement agglomerate and the deformable body.

The deformable body is advantageously made of materials having chemical and mechanical properties that are stable over time, able to be maintained within the agglomerate without deteriorating or changing over time. For example, a material of the metallic type (stainless steel or the like) can be used, or a ceramic material (alumina or the like).

This technical solution results in the further technical advantage of being able to carry out a more precise and reliable reading of the tension by means of materials of which the chemical, physical and mechanical characteristics are correctly known and foreseeable over time: the prior art within the sector, in fact, does not make it possible to obtain a predictive level of precision depending on materials of which the characteristics are or are not fully known or are potentially transient over time. The deformable body can optionally comprise, therewithin, elements that manifest viscous behaviour under load but that are dimensioned such that they confer a negligible viscous contribution with respect to the substantially resilient behaviour of the deformable body as a whole.

Such viscous elements can be made preferably using a polymer material: for example, a layer of polymer material used as a spacer (Kapton) or as an adhesive (polymer resin).

The investigation device is preferably constrained to the reinforcement.

In this way it is possible to prevent the investigation device from moving, during the step of concrete casting and in the curing period thereof, from the initial position in which it was placed, compromising a correct interpretation of the spatial correlation between the signals read by the investigation device and the surface or behavioural signs (in terms of resistance to the applied actions) demonstrated by the pre-cast segment.

In accordance with one embodiment, the deformable body has resilient behaviour, at least with regard to the stresses permissible in the structure of the cement agglomerate, and comprises two surfaces, the smaller dimension of which is greater than, or equal to, the maximum nominal diameter of a bonded material comprised in said cement agglomerate and has a substantially flattened shape with regard to two prevalent dimensions so as to obtain inside said deformable body an undisturbed zone of the tension, in which zone said at least one deformation meter is arranged.

Thanks to this technical feature, it is possible to obtain a measure of the tension within the investigation device which is not affected by the viscoelastic contributions present in the cement agglomerate of the pre-cast segment which surround the investigation device (for a more detailed and thorough description, see document IT 102016000037314).

The advantage of such a technical solution is clear, since it is possible in this way to obtain correct time-based information relating to the tension acting in a direction perpendicular to the surfaces that define the substantially flattened shape, and said information can be calculated by means of a simple combination of the three deformations detected in the undisturbed zone of the tension within the deformable body contained in the investigation device.

In particular, the deformable body preferably comprises two surfaces, the smaller dimension of which is greater than, or equal to, the maximum nominal diameter of said bonded or inert material contained within said agglomerate, specifically having a minimal transverse dimension (for example the diameter in the case of a cylindrical form) greater than or equal to the maximum nominal diameter of the bound or inert materials of the agglomerate.

It is in fact important to note that the deformable body having said substantially flattened shape with regard to two prevalent dimensions disturbs, in a negligible manner, the tension field of the tension orthogonal to the two prevalent dimensions within the body itself, thus producing an undisturbed zone of the tension not affected by viscous phenomena of first or second order. In physics, a field is a region of space, at any point of which a physical variable is defined by means of a suitable law (depending on its nature, the field will be defined as a scalar field, vector field, tensor field, etc.). Said physical variable can be a temperature (example of scalar field), a force (example of vector field: gravitational, electric or magnetic field) or a tension as in this field of application.

In this context, the term disturb in a negligible manner means that the substantially flattened shape of the deformable body comprises possible disturbances of the tension field of the tension below a predefined value. In particular, such a predefined value is equal to 10% of the local value of the tension field.

In a preferred embodiment, the at least three deformation measures oriented with respect to one another are contained in the undisturbed zone and the tension is proportional to a combination of the aforementioned three deformation measures. In addition, in the present context, substantially flattened shape preferably identifies those three-dimensional structures having a primary development along two prevalent dimensions with respect to a third: examples can be a prismatic or cylindrical or laminar model having two bases defined by the two prevalent dimensions and spaced apart from one another along the third dimension of lower height compared to the two prevalent dimensions.

The pre-cast segment preferably comprises a first, a second and a third investigation device respectively placed in opposite radial faces and in a medial zone of said arcuate structure

In this way it is possible to have an optimised analysis and monitoring of any cracks, fracturing or deformations that can occur within the aforementioned pre-cast segment.

In particular, the first and second investigation device respectively placed in said opposite radial faces make it possible to assess any change in tensions in the vicinity of the zones of movement towards one another between two segments during the step of use of the segments within the tunnel.

Such types of tensions are defined as membrane tensions and are correlated to purely surface-related interactions between the segments.

Moreover, the installation of the third investigation device in the medial zone of the arcuate structure makes it possible to compare the changes in the data provided by the first and second investigation devices so as to be able to understand how any fractures or deformations or cracks might be developing in an intermediate zone between the two radial faces.

The pre-cast segment preferably comprises at least one capacitive sensor, which is included in said arcuate structure, for detecting internal fractures of said pre-cast segment.

It is in fact known that the aforementioned pre-cast segments do not have perfectly smooth surfaces, but instead surfaces provided with microscopic protrusions that are produced intrinsically during the production processes or the successive steps of tra nsport/i nstallation.

Such surface-related protrusions ensure that the load acting between two faces moved towards one another (preferably circumferential faces) of two adjacent segments is not distributed uniformly, but at intermittent points, which in turn results in significant structural damage to the segments themselves.

Furthermore, thanks to this technical arrangement, it is possible to assess and detect "splitting" phenomena within the arcuate structure of the pre-cast segment. In this context, the splitting phenomena are related to the application of loads (permanent or temporary), which induce forces of compression in a first zone of interest and forces of tension in a second zone of interest induced by the reaction of the material to the aforementioned applied forces of compression.

As a result of the presence of the forces of tension, it is possible that the material will break, creating separating internal fracturing, and thus partially dissipating the energy accumulated by means of the formation of new internal surfaces.

By inserting capacitive sensors in these zones affected by splitting phenomena, it is therefore possible to immediately detect the potential start of said phenomenon by means of the change in the capacitance of the aforementioned sensor correlated to a change in the distance between its reinforcements.

The faces affected most by these types of stresses, and therefore defects, are advantageously the longitudinal faces that support the weight of the terrain in the outer portion, close to the outer longitudinal face and to the tunnel, and any loads applied by working machinery in the internal portion, close to the inner longitudinal face.

The at least one capacitive sensor is preferably housed in a bearing or positioning zone of jacks of a tunnel boring machine identified on a circumferential face or on an inner longitudinal face, which can be used by the tunnel boring machine for resting against during a movement step.

This technical solution makes it possible to optimise the monitoring of any splitting phenomena during or following applications of loads by a tunnel boring machine resting on a specific bearing zone. The applicant has in fact confirmed that the fracturing phenomena occur primarily in the zones in which the tunnel boring machine rests during phases of movement or in zones in which the jacks are positioned.

For example, a correspondence of the bearing zone identified on the inner longitudinal face can be defined by tracing a radial straight line segment which connects the longitudinal axis of the tunnel and the resting zone of the tunnel boring machine and by continuing it until it reaches the outer longitudinal face: such a continuation of the aforementioned segment represents the zones placed substantially in the bearing zone concerned.

In accordance with one embodiment, the at least one capacitive sensor is housed at a distance from the inner longitudinal face equal to approximately half the pitch between two positioning zones of jacks.

In this way, at least one capacitive sensor is positioned in an ideal zone in which any potential fracturing phenomena develop to a greater extent.

In fact, the applicant has confirmed that the distance at which most types of cracking of this type manifest themselves is equal to approximately 0.5 or 0.6 of the step between two consecutive zones of positioning of contact jacks of the tunnel boring machine. The pre-cast segment preferably comprises at least one inclinometer disposed in or on the arcuate structure and configured to detect variations of ovalisation of the pre-cast segment.

In this way it is furthermore possible to perform an assessment of "macroscopic" changes in inclination or orientation of the segment once installed.

Such changes can occur typically following natural phenomena, such as landslides, earthquakes, etc., or following artificial phenomena, such as the production of boreholes for tunnels in the vicinity of the pre-existing tunnel, production of foundations for building structures, etc.

It is interesting to note that the pre-cast segment produced in accordance with the present invention gives the possibility of comprising three different types of sensors dedicated to specific information:

- deformation meters, which provide local information preferably relating to membrane tensions,
- capacitive sensors, which provide local information preferably relating to the phenomena of fracturing/splitting,
- inclinometers, which provide information preferably relating to phenomena in respect of a change in inclination/orientation of the segments following structural subsidence.

It is also important to note that the most conventional monitoring procedures generally provide information relating to the level of deformation of the tunnels in analyses, but do not allow a measurement of the tensions within the concrete and do not make it possible to perform a back-analysis in order to assess the thrust acting on the terrain. The algorithms and the monitoring procedures proposed here overcome this drawback by means of the preferably simultaneous installation on the structure of two types of instrumentation and advantageously by means of the development of algorithms for ad hoc analysis.

The tunnel is advantageously equipped with stress sensors inserted within the rings. The tension sensors can be installed in production steps of the segments of the tunnel (smart segments), or possibly can be post-installed within existing structures. As already discussed, in the case of installation of the sensors in prefabrication steps, the segments equipped with sensors provide data relating to the membrane stresses induced on the tunnel also in construction steps.

At the end of the construction step of the tunnel, a set of inclinometers (in a minimum number determined on the basis of the geometry and radius of the tunnel) is mounted at the intrados of the cavity so as to describe, exhaustively, the deformative process of the cavity. The readers of the sensors will be free from influences of external ambient factors or measurement errors through a data processing algorithm developed precisely for the sensors used. The readings of the inclinometers will give the rotation of each point of the tunnel, on the basis of which the ovalisation of the tunnel over time can be calculated. Once the ovalisation is known, the flexural stresses to which the tunnel is subjected can be ascertained by means of FEM modelling of the tunnel. The stress sensors preferably embedded in the structure provide information on the state of axial stress of the tunnel segments. The inclusion of tension sensors and inclinometers makes it possible to fully reconstruct the state of compression bending of each tunnel section.

On the basis of the monitored deformation phenomenon and on the basis of the state of inner stress of the tunnel, it is possible to estimate the external loads of the terrain acting on the tunnel by means of a back-analysis procedure, working backwards until reaching the solution.

In accordance with one embodiment, the pre-cast segment comprises a first plurality of transverse inclinometers operatively interconnected and housed with predefined step along a direction transverse to the longitudinal axis and/or a second plurality of longitudinal inclinometers operatively interconnected and housed with predefined step along a direction parallel to the longitudinal axis.

In this way, it is possible to compare and monitor the development of the inclinations/orientations of the segment in more points with reference to the contributions in the transverse or longitudinal direction with respect to the axis of the tunnel.

The first plurality of transverse inclinometers and the second plurality of longitudinal inclinometers are preferably housed on a plurality of segments so as to increase the amount of data collected and the information that can be correlated to the integrity of the tunnel.

In accordance with one embodiment, the first plurality of transverse inclinometers or the second plurality of longitudinal inclinometers are positioned in or on a flexible strip.

Advantageously, when the flexible strip comprises the first plurality of transverse inclinometers it is disposed in a direction transverse to the longitudinal axis, whereas when the flexible strip comprises the second plurality of longitudinal inclinometers it is disposed in a direction parallel to the longitudinal axis.

The flexible strip is preferably fastened to portions of segments and/or portions of tunnel by means of cement mortars, polymer adhesives, bonding systems, nails or screws or similar technical solutions. in accordance with one embodiment, the flexible strip is fastened externally to the arcuate structure of the pre-cast segment by means of coupling devices.

The deformation sensors and/or meters that are mounted within the segments preferably produce an amount of data that is different depending on their various work phases, as shown in Table 1 below.

*Table 1: Sensor sampling characteristics*

|   | Phase | Samp. Freq. | Consumptio n | Duratio n | Communicatio n | Power supply |
|---|-------|-------------|--------------|-----------|----------------|--------------|
| 1 | Maturatio n | every 2 days | | 8 weeks | - | battery |
| 2 | Laying | every 2 minutes | | 2 days | LoRa 868MHz | battery |
| 3 | Waiting | every 2 days | | weeks | LoRa 868MHz | battery |
| 4 | Use | >4 times per day | 3W | years | Powerline | 230V AC |

The battery that powers the deformation sensors and/or meters in the first phase must advantageously guarantee the measurement, the conservation and the communication of the data collected in accordance with the timings described in Table 1. It is noted that the high frequency of sampling of phase 2 involves a greater consumption of energy, and therefore the sensors involved are preferably activated at the start of said phase and deactivated at the end.

In an illustrative example not being claimed in a scope of present invention, the collection of the data and the activation of the modes of the sensors are performed using the LoRa radio if the tests at the TBM and within the segment give positive results.

The aforementioned mechanism of activation is preferably implemented by means of a magnet system that activates a Reed relay, said relay being contained in a box immersed in the concrete. The system, once launched, is advantageously in a "ready" state, communicating by means of the LoRa radio.

An alternative to this system lies in the use of RFID at 13.5MHz.

The RFID readers used are preferably of the ATEX type.

In both cases discussed above, the activation is performed by bringing the "reader" reading/interrogation system towards the segment (this can be implemented from outside the tunnel just before the segments are transported in the TBM or during the use of the TBM itself, for example during the insertion of the screws for anchoring the segments).

Furthermore, the term segment in this context means a pre-cast structure comprising an arcuate structure of which the radial faces converge in a single centre or axis for the purpose of forming a ring by being adapted to be moved towards other adjacent segments.

The inventive product produced in accordance with the present invention is a tunnel ring comprising at least five pre-cast segments, which are moved towards one another in twos, on the respective opposite radial faces for forming a ring, at least three of the pre-cast segments being a pre-cast segment for a reinforced concrete tunnel, comprising an arcuate structure having a reinforcement and a cement agglomerate that is designed to cover structurally repeated annular tunnel segments for modules corresponding to a fraction of the cross section thereof, said arcuate structure comprising respective opposite radial faces that lie on surfaces radial with respect to a longitudinal axis of the tunnel, respective opposite circumferential faces that lie on surfaces perpendicular to the longitudinal axis, and respective opposite longitudinal faces that lie on surfaces that are parallel to the longitudinal axis, said radial faces being adapted to be moved towards respective radial faces of adjacent segments in order to form an annular tunnel portion, said circumferential faces being adapted to be moved towards one another in order to form a linear extent of said tunnel along said longitudinal axis and an outer longitudinal face being at a greater distance than an inner longitudinal face from said longitudinal axis, placed in contact with the ground of said tunnel, wherein at least one investigation device is embedded in said arcuate structure of said pre-cast segment at a predetermined distance from at least one of said radial, circumferential or longitudinal faces.

In this way, it is possible to optimise the number of pre-cast segments equipped with investigation devices with respect to the total number of segments of the tunnel, ensuring effective monitoring of the conditions of integrity of the tunnel itself.

There are preferably eighteen sections monitored in respect of pressure for each ring, two for each segment.

The rings having instrumentation shall be positioned close to those provided in the planning step and already monitored by means of extensometers.

Eight transverse inclinometers are preferably provided per ring, with two longitudinal inclinometers per ring.

In an illustrative example not being claimed in a scope of present invention, the pre-cast segments comprising instrumentation are placed in position by the TBM in the same way as traditional segments.

The following additional requirements must be observed by the segments having instrumentation; since the boxes are in line with the segment and not with cover over the perimeter of the suction cup that moves the segments (so as to have the entire cover within the perimeter or the entire cover outside the perimeter), the electronic parts supplied by the segment having instrumentation that are fully "immersed" in the concrete do not require ATEX 3 certification. Furthermore, the boxes of the segment containing powered electronics must be watertight in order to prevent the ingress of any gases in a laying step.

The box with the electronics is preferably fully foamed inside, also so as to prevent the exit of any corrosive liquid from the discharged battery over time.

The pre-cast segments preferably comprise battery power supply systems and/or 230 V AC power supply systems.

In the case in which the power supply is provided by battery, the boxes in which house the electronic components and/or the deformation sensors and/or meters are embedded within the segment at a distance from any face of said pre-cast segment equal to at least 4 cm.

This technical solution makes it possible to guarantee the ATEX requirements. The electronics in which the processing unit, the battery, and any other electronic components are contained advantageously has dimensions equal to 50*136*120mm.

The box in which the electronics are inserted is preferably positioned vertically from the outside (cover 50x136mm) towards the inside of the segment (120mm) and runs on lateral guides within the box. The cables of the sensors enter the box and run to the point at which the connectors are positioned; in this way the maintenance and possible replacement of the board is facilitated. When the monitoring system is realised, the cover is removed and exchanged for another cover, already provided with the cable of the data bus for connection to the inclinometer on the one hand and the board inside the box on the other.

A junction box will preferably have an indicative measurement of 50 x 136 x 120mm, to be positioned also vertically, such that the power supply cable can be inserted without interference with the irons.

In an illustrative example not being claimed in a scope of present invention, in addition to the primary control unit equipped with a connection to the cellular network, 14 secondary control units are positioned in the cable duct beneath the road surface, one every 500m, in wells for connection to the tunnel above.

These control units are preferably connected by optical fibre (monomodal) and include the powerline modem.

Advantageously, the number of boxes designed to contain the electronics provided and correlated to the investigation device is four per segment, positioned in the vicinity of the radial faces of the segment and connected to one another in series. The two intermediate boxes preferably have two separate inner zones for housing electronics able to produce connections in series that physically isolate the different electronics.

In an illustrative example not being claimed in a scope of present invention, at least one box has a wired face coincident with a face of said pre-cast segment. It is advantageously possible, by means of the wired face of said at least one box, to operatively connect said box, by means of a cable, to another box contained in an adjacent pre-cast segment.

The pre-cast segments equipped with an investigation device advantageously are those on which the tunnel boring machine rests during movement phases.

The applicant has in fact confirmed that, in this way, the monitoring of the zones most frequently exposed to critical loads that might induce the formation of fracturing, cracks, deformations and structural subsidence is optimised.

The operative modes of the pre-cast segment, defining the process and method of the present invention, comprise the steps described hereinafter. The process of producing and monitoring a pre-cast segment made of cement preferably comprises providing formwork for receiving a concrete cast, housing at least one investigation device in said formwork, operatively connecting said at least one investigation device to a processing unit that is capable of processing the data collected by said at least one investigation device, carrying out said concrete casting inside said formwork by embedding said at least one investigation device to form an arcuate structure of said pre-cast segment, and monitoring the data processed by said processing unit by analysing any changes in said collected or processed data during the steps following said concrete casting.

The process of producing and monitoring a pre-cast segment made of reinforced concrete preferably comprises: providing a reinforcement inside formwork for receiving said concrete cast, fastening at least one investigation device to the reinforcement, operatively connecting the at least one investigation device to a processing unit that is capable of processing the data collected by said at least one investigation device, carrying out said concrete casting inside said formwork by embedding said at least one investigation device to form an arcuate structure of said pre-cast segment, and monitoring the data processed by said processing unit by analysing any tension changes during the steps following said concrete casting. In this way, the investigation device is inserted from the initial steps of formation of the segment and makes it possible to analyse and monitor any tension changes during the following curing steps, but also, possibly, during the steps of transport and installation of the segment within the tunnel.

In accordance with one embodiment, the production and monitoring process comprises: constraining at least one capacitive sensor to a support or to said reinforcement before carrying out said concrete casting, operatively connecting said at least one capacitive sensor to said processing unit capable of processing the data collected by said at least one capacitive sensor, and monitoring the data processed by said processing unit by analysing any structural changes detected by said at least one capacitive sensor during the steps following said concrete casting.

In this way, it is possible to analyse and monitor any presence of fracturing phenomena in the pre-cast segment.

The support is preferably a three-dimensional structure positioned on a surface identifying the casting zone and capable of acting as a stable fastening means for the capacitive sensor (for example a metal or polymer structure).

The production and monitoring process preferably comprises: constraining at least one inclinometer to the outside of said arcuate structure after a predefined curing time from when the concrete casting took place, operatively connecting said at least one inclinometer to said processing unit that is capable of processing the data collected by said at least one inclinometer, and monitoring the data processed by said processing unit by analysing any changes of inclination detected by said at least one inclinometer during the steps following said concrete casting.

In this way, it is possible to analyse and monitor any presence of phenomena of deformations and/or movements of the pre-cast segment.

In accordance with one embodiment, the production and monitoring process comprises: waiting during said curing time for said arcuate structure of said pre-cast segment, installing said pre-cast segment inside a tunnel, and monitoring the data processed by said processing unit analysing any structural changes detected. In this way, it is possible to analyse and monitor any presence of deformations, fracturing, cracks or movements of the pre-cast segment during and following its installation in a tunnel.

This operative mode thus allows effective and constant monitoring able to guarantee the integrity of the analysed segments and a timely responsiveness in the event of the presence of dangerous structural defects.

The method for producing and monitoring the tunnel preferably comprises: making a hole in a terrain, installing pre-cast segments on opposite radial faces by moving them towards one another in twos, so as to form a tunnel ring, at least one of said pre-cast segments comprising an investigation device such that, once connected to a processing unit, the processed data can be monitored during and after the installation of said pre-cast segments in said tunnel.

In this way the applicant has confirmed that a tunnel in which at least one of the installed pre-cast segments allows the analysis and monitoring of detected structural data can be constructed in a safe manner.

The method preferably comprises: using a tunnel boring machine to form said hole for said tunnel and to install said pre-cast segments, at least one of said pre-cast segments comprising an investigation device, connected to a processing unit, and monitoring the data processed during and after the installation of said pre-cast segments in said tunnel and during and after the advancing of the tunnel boring machine so that it touches said pre-cast segments, at least one of said pre-cast segments comprising said investigation device.

In this way, it is possible to analyse and monitor the structural integrity of the pre-cast segments including at least one investigation device during the tunnelling steps by means of tunnel boring machine and during the steps of installation of the segments with one another. The applicant has confirmed that these steps are particularly critical with regard to the structural integrity of the segments and therefore constitute moments of particular significance for analysis and monitoring. The method advantageously comprises advancing the tunnel boring machine along a longitudinal axis of the tunnel by resting it against at least one pre-cast segment, and monitoring the possible variations in the processed data during and after the steps of resting of the tunnel boring machine.

The applicant has confirmed that the thrust phase produced by the tunnel boring machine on the segments during the movements represents a moment in which very high loads are applied, which often can induce critical damage to the pre-cast segments. In this sense, analysing and monitoring the parameters provided by the investigation devices and sensors contained in the pre-cast segment having the features of the present invention makes it possible to optimise the monitoring of the structure for the purpose of detecting, in a timely manner, any loss of integrity of a portion of the tunnel.

Brief description of the drawings

The features and advantages of the invention will become clearer from the detailed description of a preferred embodiment thereof, provided by way of non-limiting example, and from the accompanying drawings, in which

- fig. 1 is a perspective view of a pre-cast segment for a tunnel made of reinforced concrete,
- fig. 2 is a perspective view of a reinforcement comprised in the pre-cast segment of fig. 1,

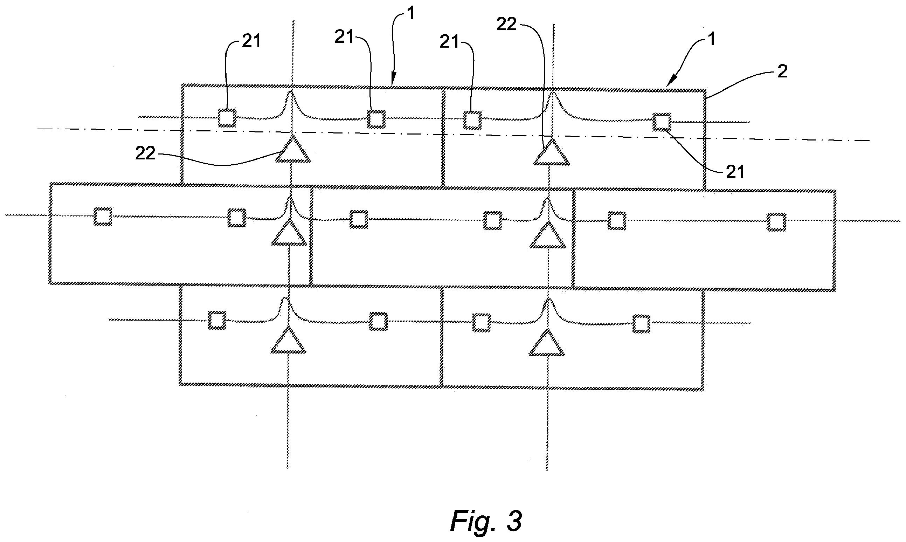

- fig. 3 is a view from beneath of a plurality of pre-cast segments disposed adjacently to one another within a tunnel,

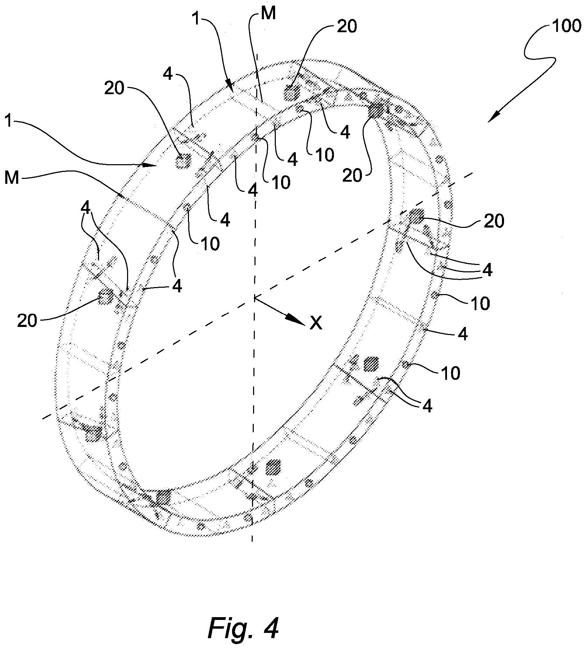

- fig. 4 is a perspective view of a tunnel ring composed of a plurality of pre-cast segments moved towards one another in twos on radial faces,
- fig. 5 is a perspective view of an investigation device and of the deformations and tensions correlated thereto,
- fig. 6 is a perspective view of an investigation device that can be associated with a structure of an agglomerate.

Preferred embodiment of the invention

In the drawings, reference sign 1 indicates a pre-cast segment 1 for a reinforced concrete tunnel produced in accordance with the present invention and designed to carry out a process and a method for producing and monitoring the pre-cast segment and a tunnel comprising the aforementioned pre-cast segment.

The pre-cast segment 1 preferably comprises an arcuate structure 2 having a reinforcement 3 and a cement agglomerate.

In accordance with one embodiment, the pre-cast segment 1 is designed to cover structurally repeated annular tunnel segments with modules corresponding to a fraction of the cross section thereof. The arcuate structure 2 comprises respective opposite radial faces 2a, 2b that lie on surfaces radial with respect to a longitudinal axis X of the tunnel, respective opposite circumferential faces 2c, 2d that lie on surfaces perpendicular to the longitudinal axis X, and respective opposite longitudinal faces 2e, 2f that lie on surfaces parallel to the longitudinal axis X.

The radial faces 2a, 2b are advantageously moved towards respective radial faces of adjacent segments in order to form an annular tunnel portion, said circumferential faces 2c, 2d being adapted to be moved towards one another in order to form a linear extent of said tunnel along said longitudinal axis X and an outer longitudinal face 2f being at a greater distance than an inner longitudinal face 2e from said longitudinal axis X, which inner longitudinal face is placed in contact with the ground of said tunnel, wherein at least one investigation device 4 is embedded in said arcuate structure 2 of said pre-cast segment 1 at a predetermined distance D from at least one of said radial 2a, 2b, circumferential 2c, 2d or longitudinal 2e, 2f faces.

The arcuate structure preferably has a length between 2 and 6 metres, a thickness between 30 and 80 centimetres, and a width between 2 and 3 metres.

In accordance with one embodiment, the distance D (not shown in the drawings) can be defined arbitrarily by a user on the basis of the specific requirements. The distance D is advantageously defined by the radial faces and is between 0 and 20 cm.

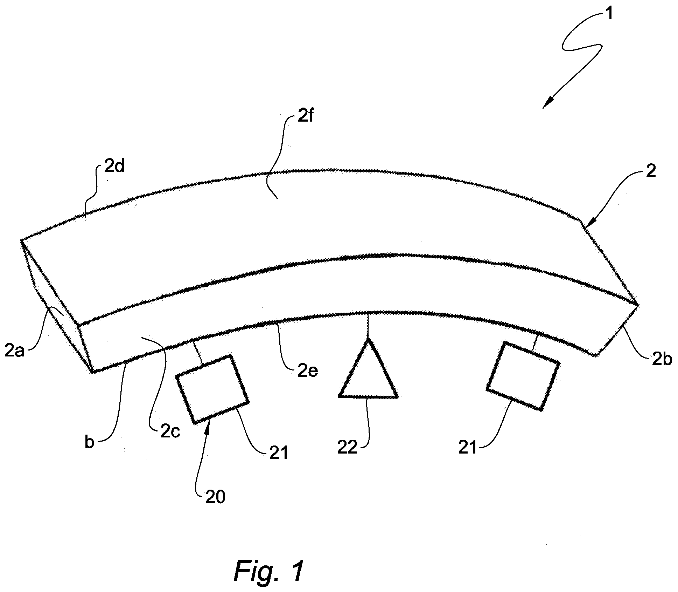

With reference to fig. 1, the pre-cast segment 1 preferably has opposite circumferential faces 2c, 2d of substantially curved trapezoidal shape, whereas the remaining faces 2a, 2b, 2e, 2f are substantially rectangular in shape.

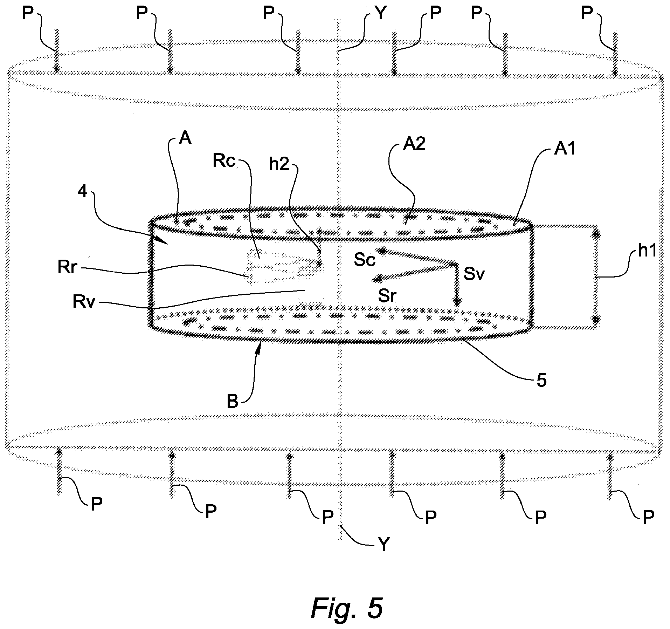

In accordance with one embodiment, the investigation device 4 comprises a deformable body 5, in which there is arranged at least one deformation meter $P_i$ configured to detect at least three deformation measures E1, E2, E3 oriented with respect to one another, such that a tension SYY within said investigation device 4 is proportional to a combination of the three deformation measures E1, E2, E3 (see fig. 5).

This deformation meter $P_i$ is preferably a device comprising at least a trio of electrical or optical extensometers.

In a preferred embodiment, the deformation meter $P_i$ comprises at least a trio of resistors (strain-gauge, piezoresistive, etc.), the deformation of which is easily detectable by monitoring the changes in the electrical resistances. Alternatively or additionally, the deformation meter $P_i$ can comprise a capacitive capacitor, the deformation of which can be easily detected by monitoring changes in the capacitance. Such embodiments are intended to be exemplary and non-limiting and can be easily and in a commonplace manner adapted, or exchanged for similar deformation sensors by persons skilled in the art for the purpose of realising the objectives of the invention

In one preferred embodiment the deformation meter $P_i$ comprises a trio of deformation sensors R1, R2, R3, oriented individually in accordance with a predetermined tri-axis system and able to measure three deformations E1, E2, E3, on the basis of which the tension SYY is calculated by way of combination, said tension acting in an undisturbed zone A2 and being proportional to the combination of the three deformation measures E1, E2, E3 and therefore solely to the external actions applied (for example a load P) and not being influenced by viscous phenomena generated within the deformable body 5 (see fig. 5).

In particular, the at least one deformation meter Pi comprises three deformation sensors Rv, Rr, Rc, individually oriented in accordance with a predetermined orthogonal trio and able to measure three deformations oriented orthogonally with respect to one another Ev, Er, Ec contained in the undisturbed zone A2, and the tension SYY is proportional to a combination of said three deformation measures oriented orthogonally with respect to one another Ev, Er, Ec. For example, in the hypothesis of axial-symmetrical form both around a portion of the cement agglomerate surrounding the investigation device, and of the investigation device 4 and with a load P applied along an reference axis, the orthogonal trio defined in accordance with the universal vertical, radial and circumferential cylindrical coordinates v, r, c also defines the orientations of the three sensors of vertical, radial and circumferential deformation Rv, Pr, Rc, of the three co-respective vertical, radial and circumferential deformations Ev, Er, Ec and of the three vertical, radial and circumferential tensions Sv, Sr, Sc respectively (see fig. 5). In addition, in the axial-symmetrical approximation used, the three vertical, radial and circumferential deformations Ev, Er, Ec are different from zero whereas the cutting deformations Erv, Erc, Evc are zero or negligible. This means that the calculation of the axial tension SYY, coincident with the vertical tension Sv, is simplified by applying the resilient linear constitutive bond of the material from which the deformable body 5 is made, in the hypothesis of an axial-symmetrical state according to the following equation (1):

$$\begin{Bmatrix} SXX \\ SYY \\ SZZ \\ SXY \end{Bmatrix} = C \begin{bmatrix} 1-v & v & v & 0 \\ v & 1-v & v & 0 \\ v & v & 1-v & 0 \\ 0 & 0 & 0 & G/C \end{bmatrix} \cdot \begin{Bmatrix} EXX \\ EYY \\ EZZ \\ EXY \end{Bmatrix} \qquad (1)$$

If it is desired to abandon the hypothesis of axial symmetry, it is necessary to apply the full constitutive relation of the material using the six components of deformation and of tension by using a more complex equation, but yielding substantially identical results (see equation (2)).

$$\begin{Bmatrix} SXX \\ SYY \\ SZZ \\ SXY \\ SXZ \\ SYZ \end{Bmatrix} = C \begin{bmatrix} 1-v & v & v & 0 & 0 & 0 \\ v & 1-v & v & 0 & 0 & 0 \\ v & v & 1-v & 0 & 0 & 0 \\ 0 & 0 & 0 & G/C & 0 & 0 \\ 0 & 0 & 0 & 0 & G/C & 0 \\ 0 & 0 & 0 & 0 & 0 & G/C \end{bmatrix} \cdot \begin{Bmatrix} EXX \\ EYY \\ EZZ \\ EXY \\ EXZ \\ EYZ \end{Bmatrix} \qquad (2)$$

where:

$$C = \frac{E}{(1+v)(1-2v)} \qquad G = \frac{E}{2(1+v)}$$

where v is the Poisson's ratio and E is the Young's modulus.

Both hypotheses give the equation (3):

$$Sv = SYY = C\big[vEXX + (1-v)EYY + vEZZ\big] \qquad (3)$$

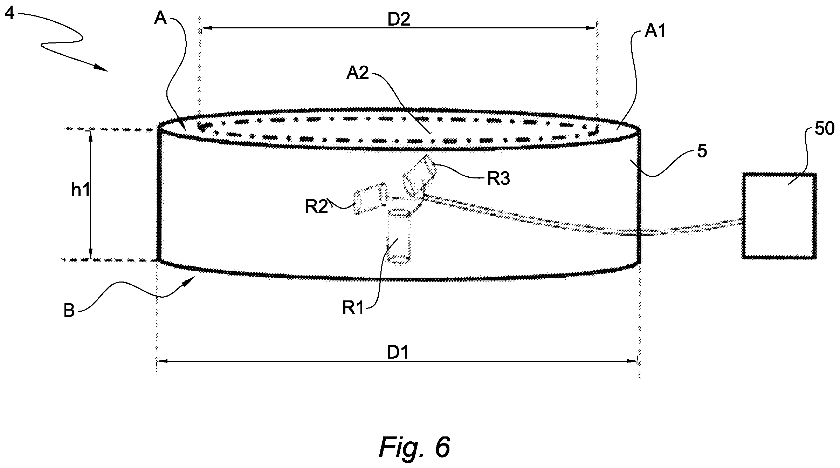

Thanks to the investigation device 4 and the above-mentioned features thereof, even in the case in which the model of the device is not axially symmetrical it is possible to define an undisturbed zone A2, within the deformable body 5, in which the tension Sv is proportional solely to the external actions applied and is not influenced by viscous phenomena produced within the deformable body 5 or by deviations of the force lines (see figs. 5 and 6).

In this case the calculations necessary to obtain the value of the tension require the use of the full constitutive bond of the material from which the deformable body 5 is made, as described above.

In one preferred embodiment, in the case in which the applied external load P is constant, the above-mentioned combination of the equation 3 is constant and this implies that the value of SYY is also constant.

The investigation device 4 is preferably fastened to the reinforcement 3 (for example by means of bonding, welding, gluing, etc.).

In accordance with one embodiment, the deformable body 5 has resilient behaviour, at least with regard to the stresses permissible in said structure of said cement agglomerate.

The deformable body 5 advantageously comprises two surfaces A, B, the smaller dimension of which is greater than, or equal to, the maximum nominal diameter of a bonded material comprised in said cement agglomerate.

The deformable body 5 preferably has a substantially flattened shape with regard to two prevalent dimensions so as to obtain inside said deformable body 5 an undisturbed zone A2 of said tension SYY, in which zone said at least one deformation meter Pi is arranged.

With reference to figs. 5 and 6 and as a function of the ratio R between the minimum dimension of the surfaces A and B (also called the base surfaces) and a height h1 of the deformable body 5, the disturbed zone is spatially confined to a more or less extensive portion of the surfaces A and B. Such disturbances, however, only concern an outer cylindrical crown, referred to as A1, of the deformable body 5 for an extension equal to a fraction of the radius (in the case of the cylindrical device shown in figs. 5 and 6, such extension is approximately 1/3 of the radius of the deformable body 5).

It is therefore possible to identify a zone within the deformable body 5, i.e. said undisturbed zone A2, which remains undisturbed by such viscous phenomena of first and second order and which is therefore subject to a tension Sv=SYY orthogonal to the faces A and B and proportional solely to the agents acting from outside (for example the load P as shown in fig. 5).

In particular, the tension Sv=SYY orthogonal to the faces A and B is proportional to a combination of a plurality of deformations comprised in the aforementioned undisturbed zone A2.

With reference to fig. 5, the undisturbed zone A2 is preferably identifiable with a cylinder within the deformable body 5, having a base of diameter D2 equal to approximately 20mm.

Said at least one deformation meter Pi is preferably positioned at a distance h2 from at least one of said two surfaces A, B (see fig. 5).

Furthermore preferably, the distance h2 is greater than, or equal to, the dimension of the larger gas bubble possibly present in the agglomerate. In fact, such a positioning of the aforementioned deformation meter R1 allows the material from which the deformable body 5 is made to reduce the local disturbance caused to the measurement by the gas bubbles present in the agglomerate.

In accordance with one embodiment, the deformation meter Ri is disposed equidistantly between the two faces A and B.

The deformation meter Pi is alternatively disposed asymmetrically within said deformable body, but maintaining the minimum distance between a part thereof and the face A or B greater than said dimension of the larger gas bubble.

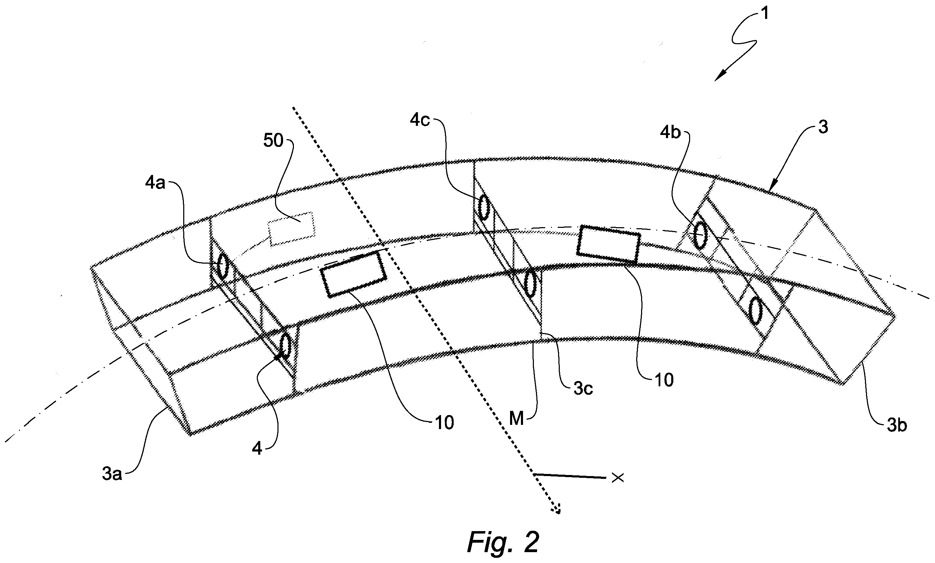

With reference to fig. 2 the pre-cast segment 1 preferably comprises a first, a second and a third investigation device 4a, 4b, 4c respectively placed in opposite radial faces 2a, 2b, 2c and in a medial zone M of the arcuate structure 2.

The first, second and third investigation devices 4a, 4b, 4c are preferably arranged along a radial alignment with the longitudinal axis X of the tunnel, and the first and second device are substantially parallel to the respective radial face in which they are housed.

The first, second and third investigation devices 4a, 4b, 4c are advantageously pluralities of investigation devices housed respectively in opposite radial faces 2a, 2b and in the medial zone M of the arcuate structure 2.

In an illustrative example not being claimed in a scope of present invention, the investigation device 4, the first, the second or the third investigation device 4a, 4b, 4c, are operatively connected to a processing unit 50 that is capable of processing the data collected by said investigation devices. This operative connection can be established by means of cables (preferably equipped with watertight connectors) or by means of wireless data transfer systems.

Furthermore, the investigation device and/or the processing unit can be powered by means of a battery or by means of an electrical connection to an external power supply line.

Such connections are established by means of cables and connectors guaranteed in accordance with standard IP68 and satisfying the ATEX parameters.

The pre-cast segment 1 preferably comprises at least one capacitive sensor 10, included in the arcuate structure 2, for detecting internal fractures of the pre-cast segment 1.

Persons skilled in the art will be able to identify the type of capacitive sensors available on the market that can be best applied for the needs of the present invention.

In accordance with one preferred embodiment, the capacitive sensor 10 is operatively connected to the processing unit 50 that is capable of processing the data from said sensor. This operative connection can be established by means of cables (preferably equipped with watertight connectors) or by means of wireless data transfer systems.

Furthermore, the capacitive sensor and/or the processing unit can be powered by means of a battery or by means of an electrical connection to an external power supply line.

In accordance with one embodiment, the at least one capacitive sensor 10 is housed in a resting zone S1 identified on a circumferential face 2c, 2d or on the inner longitudinal face 2e usable by a tunnel boring machine to rest against during a movement phase.

The at least one capacitive sensor 10 is preferably housed within the arcuate structure at a distance F (not shown in the drawings) from the inner longitudinal face 2e equal to approximately half the pitch between two consecutive positioning zones S1, S2 of two contact jacks of said tunnel boring machine.

The applicant has in fact demonstrated that applications of loads from the tunnel boring machine aimed at producing the movement by means of resting on said surfaces of a circumferential face 2c, 2d or on the inner longitudinal face 2e can induce phenomena of fracturing within the segment itself and in particular at the jacks of the tunnel boring machine used as bearing points. In accordance with one embodiment, the step between the two positioning zones S1, S2 is around 1.20 m, and therefore the distance F is equal to approximately 0.60 m.

In accordance with one embodiment, the pre-cast segment 1 comprises at least one inclinometer 20 arranged in or on the arcuate structure 2 and configured to detect variations of ovalisation of the pre-cast segment 1.

The inclinometer 20 preferably comprises a watertight box inside which there is housed an inclination sensor.

The inclinometer 20 is advantageously fastened from the outside to the arcuate structure 2 by means of a bracket or similar technical solutions advantageously making it possible to fix said inclinometer with a preferred orientation.

In accordance with one embodiment, the inclinometer 20 is constrained to the inner longitudinal face 2e, which extends towards the interior of the tunnel.

Persons skilled in the art will be able to identify the type of inclinometer available on the market that can be best applied to the needs of the present invention.

In accordance with one preferred embodiment, the inclinometer 20 is operatively connected to the processing unit 50 able to process the data received from said inclinometer. This operative connection can be established by means of cables (preferably equipped with watertight connectors) or by means of wireless data transfer systems.

Furthermore, the inclinometer 20 and/or the processing unit can be powered by means of a battery or by means of an electrical connection to an external power supply line.

The pre-cast segment 1 preferably comprises a first plurality of transverse inclinometers 21 operatively interconnected and housed with a predefined step along a direction transverse to the longitudinal axis X and/or a second plurality of longitudinal inclinometers 22 operatively interconnected and housed with a predefined step along a direction parallel to said longitudinal axis X.

In accordance with one embodiment, the first plurality of transverse inclinometers 21 or the second plurality of longitudinal inclinometers 22 are positioned in or on a flexible strip.

The inventive product produced in accordance with the present invention is a tunnel ring 100 comprising at least five pre-cast segments, which are moved towards one another in twos, on the respective opposite radial faces 2a, 2b for forming a ring, at least three of the pre-cast segments being formed as said pre-cast segment 1 that is designed to cover structurally repeated annular tunnel segments for modules corresponding to a fraction of the cross section thereof. The arcuate structure 2 comprises respective opposite radial faces 2a, 2b that lie on surfaces radial with respect to the longitudinal axis X of the tunnel, respective opposite circumferential faces 2c, 2d that lie on surfaces perpendicular to the longitudinal axis X, and respective opposite longitudinal 2e, 2f faces that lie on surfaces that are parallel to the longitudinal axis X. The radial faces 2a, 2b are advantageously moved towards respective radial faces of adjacent segments in order to form an annular tunnel portion, said circumferential faces 2c, 2d being adapted to be moved towards one another in order to form a linear extent of said tunnel along said longitudinal axis X and an outer longitudinal face 2f being at a greater distance than an inner longitudinal face 2e from said longitudinal axis X, placed in contact with the ground of said tunnel, wherein at least one investigation device 4 is embedded in said arcuate structure 2 of said pre-cast segment 1 at a predetermined distance D from at least one of said radial 2a, 2b, circumferential 2c, 2d or longitudinal 2e, 2f faces. The operating modes of the pre-cast segment, defining the process and method of the present invention, comprises the steps described hereinafter.

The process 200 for producing and monitoring a pre-cast segment 1 made of cement preferably comprises providing formwork for receiving a concrete cast, housing at least one investigation device 4 in said formwork, operatively connecting said at least one investigation device 4 to a processing unit 50 that is capable of processing the data collected by said at least one investigation device 4, carrying out said concrete casting inside said formwork by embedding said at least one investigation device 4 to form an arcuate structure 2 of said pre-cast segment 1, and monitoring the data processed by said processing unit 50 by analysing any changes in said collected or processed data during the steps following said concrete casting.

In this context, the term cement means both the structures in reinforced concrete (and therefore equipped with reinforcement) and reinforced concretes (and therefore comprising metal or ceramic fibres therewithin capable of increasing the mechanical characteristics of the concrete itself).

The process 200 of producing and monitoring a pre-cast segment 1 made of reinforced concrete preferably comprises: providing a reinforcement 3 within formwork for receiving a concrete cast, fastening at least one investigation device 4 to the reinforcement 3, operatively connecting the at least one investigation device 4 to a processing unit 50

that is capable of processing the data collected by the at least one investigation device 4, carrying out said concrete casting inside said formwork by embedding the reinforcement 3 and the at least one investigation device 4 to form an arcuate structure 2 of the pre-cast segment 1, and monitoring the data processed by said processing unit 50 by analysing any tension changes during the steps following said concrete casting.

The production and monitoring process 200 preferably comprises: constraining at least one capacitive sensor 10 to a support or to the reinforcement 3 before carrying out said concrete casting, operatively connecting the at least one capacitive sensor 10 to the processing unit 50 capable of processing the data collected by said at least one capacitive sensor 10, and monitoring the data processed by said processing unit 50 by analysing any structural changes detected by said at least one capacitive sensor 10 during the steps following said concrete casting.

In accordance with one embodiment, the production and monitoring process 200 comprises constraining at least one inclinometer 20 to the outside of said arcuate structure 2 after a predefined curing time Tc from when the concrete casting took place, operatively connecting said at least one inclinometer 20 to said processing unit 50 that is capable of processing the data collected by said at least one inclinometer 20, and monitoring the data collected or processed by said processing unit 50 by analysing any changes of inclination detected by said at least one inclinometer 20 during the steps following said concrete casting.

The curing time Tc is preferably comprised within the period of 8 weeks.

In accordance with one embodiment, the production and monitoring process 200 comprises: waiting during said curing time Tc of the arcuate structure 2 of the pre-cast segment 1, installing the pre-cast segment 1 inside a tunnel, and monitoring the data processed by said processing unit 50 by analysing any structural changes detected.

The method 300 for producing and monitoring a tunnel preferably comprises: making a hole in a terrain, installing pre-cast segments on opposite radial faces 2a, 2b by moving them towards one another in twos, so as to form a tunnel ring, at least one of said pre-cast segments comprising an investigation device 4 such that, once connected to a processing unit 50, the processed data can be monitored during and after the installation of said pre-cast segments in said tunnel.

In an illustrative example not being claimed in a scope of present invention, the method 300 comprises using a tunnel boring machine to make the hole for the tunnel and to install the pre-cast segments, at least one of said pre-cast segments 1 comprising an investigation device 4.

The investigation device 4 is then connected to a processing unit 5 by advancing said tunnel boring machine so that it touches said pre-cast segments, at least one of said pre-cast segments 1 comprising said investigation device 4, and monitoring the data processed during and after the installation of the pre-cast segments in said tunnel. The data operatively transferred from the investigation device 4 and processed by the processing unit 5 is of the ASCII or raw type or the like, allowing said data to be processed and displayed by said processing unit in accordance with force/deformation ratio models or the like, known within the field of study of cement structures.

In an illustrative example not being claimed in a scope of present invention, the tunnel boring machine is moved along the tunnel, pushing on a bearing or positioning zone of jacks identified on a circumferential face 2c, 2d or on the inner longitudinal face 2e of the aforementioned pre-cast segment 1.

The method 300 preferably comprises advancing the tunnel boring machine along a longitudinal axis X of the tunnel by resting it against at least one pre-cast segment 1 and monitoring any changes in the data processed during and after the resting phases of the tunnel boring machine.

In this way, it is possible to evaluate, in real time, any changes in structural responses provided by the pre-cast segment 1 as a result of the formation of fracturing, cracks or deformations.


**Claims**

1. Pre-cast segment (1) for a reinforced concrete tunnel, comprising an arcuate structure (2) having a reinforcement (3) and a cement agglomerate that is designed to cover structurally repeated annular tunnel segments for modules corresponding to a fraction of the cross section thereof, said arcuate structure (2) comprising respective opposite radial faces (2a, 2b) that lie on planes that are angularly spaced apart from one another and passing through a longitudinal axis (X) of the tunnel, respective opposite circumferential faces (2c, 2d) that lie on surfaces perpendicular to said longitudinal axis (X) and are spaced apart along said longitudinal axis, respective opposite longitudinal faces (2e, 2f) that lie on surfaces that are parallel to said longitudinal axis (X), said radial faces (2a, 2b) being adapted to be moved towards respective radial faces of adjacent segments in order to form an annular tunnel portion, said circumferential faces (2c, 2d) being adapted to be moved towards respective circumferential faces of adjacent segments in order to form a linear extent of said tunnel along said longitudinal axis (X) and an outer longitudinal face (2f), being at a greater distance than an inner longitudinal face (2e) from said longitudinal axis (X), is placed in contact with the ground of said tunnel, wherein at least one investigation device (4) is embedded in said arcuate structure (2) of said pre-cast segment (1) at a predetermined distance (D) from at least one of said radial (2a, 2b), circumferential (2c, 2d) or longitudinal (2e, 2f) faces, so as to detect predetermined structural parameters, said investigation device (4) comprises a deformable body (5) in which at least one deformation meter (Ri) is arranged, **characterised in that** said deformation body (5) is configured to detect at least three deformation measures (E1, E2, E3) oriented with respect to one another, such that a tension (SYY) within said investigation device (4) is proportional to a combination of said three deformation measures (E1, E2, E3) and **in that** said deformable body (5) has purely resilient behaviour, at least with regard to the stresses permissible in said structure of said cement agglomerate.

2. Pre-cast segment (1) according to the preceding claim, wherein said deformable body (5) comprises two surfaces (A, B), the smaller dimension of which is greater than or equal to the maximum nominal diameter of a bonded material comprised in said cement agglomerate and has a substantially flattened shape with regard to two prevalent dimensions so as to obtain inside said deformable body (5) an undisturbed zone (A2) of said tension (SYY), in which zone said at least one deformation meter (Ri) is arranged.

3. Pre-cast segment (1) according to any one of the preceding claims, comprising

    o a first, a second and a third investigation device (4a, 4b, 4c) respectively placed in said opposite radial faces (2a, 2b) and in a medial zone (M) of said arcuate structure (2).

4. Pre-cast segment (1) according to any one of the preceding claims, comprising

    o at least one capacitive sensor (10), which is included in said arcuate structure (2), for detecting internal fractures of said pre-cast segment (1).

5. Pre-cast segment (1) according to claim 4, wherein

    o said at least one capacitive sensor (10) is housed in proximity of an abutment zone (S1) identified on a circumferential face (2c, 2d) or on said inner longitudinal face (2e), which can be used by a tunnel boring machine for resting against during a movement step.

6. Pre-cast segment (1) according to claim 5, wherein

    o said at least one capacitive sensor (10) is housed at a distance (F) from said inner longitudinal face (2e) that is equal to approximately half the pitch between two consecutive positioning zones (S1, S2) of two contact jacks of said tunnel boring machine.

7. Pre-cast segment (1) according to any one of the preceding claims, comprising

    o at least one inclinometer (20) arranged in or on said arcuate structure (2) and designed to detect variations in the ovalisation of said pre-cast segment (1).

8. Pre-cast segment (1) according to claim 7, comprising

    o a first plurality of transverse inclinometers (21) that are operatively interconnected and housed with a predefined step in a direction transverse to said longitudinal axis (X), and/or
    o a second plurality of longitudinal inclinometers (22) that are operatively interconnected and housed with a predefined pitch in a direction parallel to said longitudinal axis (X).

9. Pre-cast segment (1) according to claim 8, wherein

    ∘ said first plurality of transverse inclinometers (21) or said second plurality of longitudinal inclinometers (22) are positioned in or on a flexible strip.

10. Tunnel ring (100) comprising

    ∘ at least five pre-cast segments, which are moved towards one another in twos, on the respective opposite radial faces (2a, 2b) for closing said ring,
    ∘ at least three of said pre-cast segments being formed according to the features of the preceding claims.

11. Method (200) for producing and monitoring a pre-cast segment (1) according to any of the preceding claims made of cement, comprising

◦providing formwork for receiving a concrete cast,
◦housing at least one investigation device (4) in said formwork,
◦operatively connecting said at least one investigation device (4) to a processing unit (50) that is capable of processing the data collected by said at least one investigation device (4),
◦carrying out said concrete casting inside said formwork by embedding said at least one investigation device (4) to form said arcuate structure (2) of said pre-cast segment (1), and
◦monitoring the data processed by said processing unit (50) by analysing any changes in said collected or processed data during the steps following said concrete casting.

12. Production and monitoring method (200) according to claim 11, comprising

◦providing a reinforcement (3) inside formwork for receiving said concrete cast,
◦fastening at least one investigation device (4) to said reinforcement (3),
◦operatively connecting said at least one investigation device (4) to a processing unit (50) that is capable of processing the data collected by said at least one investigation device (4),
◦carrying out said concrete casting inside said formwork by embedding said reinforcement (3) and said at least one investigation device (4) in order to form an arcuate structure (2) of said pre-cast segment (1), and
◦monitoring the data processed by said processing unit (50) by analysing any changes in tension during the steps following said concrete casting.

13. Production and monitoring method (200) according to either claim 11 or claim 12, comprising

◦constraining at least one capacitive sensor (10) to a support or to said reinforcement (3) before carrying out said concrete casting,
◦operatively connecting said at least one capacitive sensor (10) to said processing unit (50) capable of processing the data collected by said at least one capacitive sensor (10), and
◦monitoring the data processed by said processing unit (50) by analysing any structural changes detected by said at least one capacitive sensor (10) during the steps following said concrete casting.

14. Production and monitoring method (200) according to any one of claims 11 to 13, comprising

◦constraining at least one inclinometer (20) to the outside of said arcuate structure (2) after a predefined curing time (Tc) from when the concrete casting took place,
◦operatively connecting said at least one inclinometer (20) to said processing unit (50) that is capable of processing the data collected by said at least one inclinometer (20), and
◦monitoring the data processed by said processing unit (50) by analysing any changes of inclination detected by said at least one inclinometer (20) during the steps following said concrete casting.

15. Production and monitoring method (200) according to any one of the preceding claims 11 to 14, comprising

◦waiting during said curing time (Tc) for said arcuate structure (2) of said pre-cast segment (1),
◦installing said pre-cast segment (1) inside a tunnel, and
◦monitoring the data collected or the data processed by said processing unit (50) analysing any structural changes detected.

**Patentansprüche**

1. Vorgefertigtes Segment (1) für einen Stahlbetontunnel, das eine bogenförmige Struktur (2) mit einer Bewehrung (3) und einem Zementagglomerat aufweist, das bestimmt ist, um sich strukturell wiederholende ringförmige Tunnelsegmente für Module zu überdecken, die einem Teil ihres Querschnitts entsprechen, wobei die bogenförmige Struktur (2) jeweils gegenüberliegende radiale Flächen (2a, 2b) aufweist, die auf Ebenen liegen, die winkelförmig voneinander beabstandet sind und durch eine Längsachse (X) des Tunnels verlaufen, jeweils gegenüberliegende Umfangsflächen (2c, 2d) aufweist, die auf Flächen senkrecht zur Längsachse (X) liegen und entlang der Längsachse voneinander beabstandet sind, jeweils gegenüberliegende Längsflächen (2e, 2f) aufweist, die auf Flächen liegen, die parallel zur Längsachse (X) sind, wobei die radialen Flächen (2a, 2b) angepasst sind, um in Richtung der jeweiligen radialen Flächen benachbarter Segmente bewegt zu werden, um einen ringförmigen Tunnelabschnitt zu formen, wobei die Umfangsflächen (2c, 2d) angepasst sind, um in Richtung der jeweiligen Umfangsflächen benachbarter Segmente bewegt zu werden, um eine lineare Erstreckung des Tunnels entlang der Längsachse (X) zu formen, und eine äußere Längsfläche (2f), die einen größeren Abstand als eine innere Längsfläche (2e) von der Längsachse (X) aufweist, in Kontakt mit dem Boden des Tunnels angeordnet ist, wobei zumindest eine Untersuchungsvorrichtung (4) in der bogenförmigen Struktur (2) des vorgefertigten Segments (1) in einem vorbestimmten Abstand (D) von zumindest einer der Radialflächen (2a, 2b), Umfangsflächen (2c, 2d) oder Längsflächen (2e, 2f) eingebettet ist, um vorgegebene Strukturparameter zu erfassen, wobei die Untersuchungsvorrichtung (4) einen verformbaren Körper (5) aufweist, in dem zumindest ein Verformungsmesser (Ri) angeordnet ist, **dadurch gekennzeichnet, dass** der Verformungskörper (5) eingerichtet ist, um zumindest drei zueinander ausgerichtete Verformungsmaße (E1, E2, E3) zu erfassen, so dass eine Spannung (SYY) innerhalb der Untersuchungsvorrichtung (4) proportional zu einer Kombination der drei Verformungsmaße (E1, E2, E3) ist, und dadurch, dass der verformbare Körper (5) ein rein elastisches Verhalten zumindest im Hinblick auf die in der Struktur des Zementagglomerats zulässigen Spannungen aufweist.

2. Vorgefertigtes Segment (1) nach dem vorhergehenden Anspruch, wobei der verformbare Körper (5) zwei Flächen (A, B) aufweist, deren kleinere Abmessung größer oder gleich dem maximalen Nenndurchmesser eines Verbundmaterials ist, das im Zementagglomerat enthalten ist, und im Hinblick auf zwei vorherrschende Abmessungen eine im Wesentlichen abgeflachte Form aufweist, um im Inneren des verformbaren Körpers (5) eine ungestörte Zone (A2) der Spannung (SYY) zu erhalten, in der der zumindest eine Verformungsmesser (Ri) angeordnet ist.

3. Vorgefertigtes Segment (1) nach einem der vorhergehenden Ansprüche, umfassend:

   - eine erste, eine zweite und eine dritte Untersuchungsvorrichtung (4a, 4b, 4c), die jeweils in den gegenüberliegenden radialen Flächen (2a, 2b) und in einer mittleren Zone (M) der bogenförmigen Struktur (2) angeordnet sind.

4. Vorgefertigtes Segment (1) nach einem der vorhergehenden Ansprüche, umfassend:

   - zumindest einen kapazitiven Sensor (10), der in der bogenförmigen Struktur (2) enthalten ist, um innere Risse des vorgefertigten Segments (1) zu erfassen.

5. Vorgefertigtes Segment (1) nach Anspruch 4, wobei

   - zumindest ein kapazitiver Sensor (10) in der Nähe einer auf einer Umfangsfläche (2c, 2d) oder auf der inneren Längsfläche (2e) gekennzeichneten Anlagezone (81) aufgenommen ist, die von einer Tunnelbohrmaschine zur Anlage während eines Bewegungsschrittes genutzt werden kann.

6. Vorgefertigtes Segment (1) nach Anspruch 5, wobei

   - zumindest ein kapazitiver Sensor (10) in einem Abstand (F) von der inneren Längsfläche (2e) aufgenommen ist, der etwa der Hälfte des Abstands zwischen zwei aufeinanderfolgenden Positionierungszonen (81, 82) zweier Kontaktzylinder der Tunnelbohrmaschine entspricht.

7. Vorgefertigtes Segment (1) nach einem der vorhergehenden Ansprüche, umfassend:

   - zumindest einen Neigungsmesser (20), der in oder an der bogenförmigen Struktur (2) angeordnet ist, und bestimmt ist, um Änderungen in der Ovalisierung des vorgefertigten Segments (1) zu erfassen.

8. Vorgefertigtes Segment (1) nach Anspruch 7, umfassend:

   - eine erste Mehrzahl von Querneigungsmessern (21), die miteinander wirkverbunden und mit einer vordefinierten Stufe in einer Richtung quer zur Längsachse (X) aufgenommen sind, und/oder
   - eine zweite Mehrzahl von Längsneigungsmessern (22), die miteinander wirkverbunden und mit einem vordefinierten Abstand in einer Richtung parallel zur Längsachse (X) aufgenommen sind.

9. Vorgefertigtes Segment (1) nach Anspruch 8, wobei

- die erste Mehrzahl von Querneigungsmessern (21) oder die zweite Mehrzahl von Längsneigungsmessern (22) in oder auf einem flexiblen Streifen positioniert sind.

10. Tunnelring (100), umfassend:

- zumindest fünf vorgefertigte Segmente, die jeweils zu zweit an den jeweils gegenüberliegenden Radialflächen (2a, 2b) aufeinander zubewegt werden, um den Ring zu schließen,
- zumindest drei der vorgefertigten Segmente, die gemäß den Merkmalen der vorhergehenden Ansprüche ausgebildet sind.

11. Verfahren (200) zum Herstellen und Überwachen eines aus Zement vorgefertigten Segments (1) nach einem der vorhergehenden Ansprüche, umfassend:

- Vorsehen einer Schalung zum Aufnehmen eines Betongusses,
- Unterbringen der zumindest einen Untersuchungsvorrichtung (4) in der Schalung,
- Wirkverbinden des zumindest einen Untersuchungsgeräts (4) mit einer Verarbeitungseinheit (50), die in der Lage ist, die von zumindest einem Untersuchungsgerät (4) gesammelten Daten zu verarbeiten,
- Ausführen des Betongießens innerhalb der Schalung durch Einbetten der zumindest einen Untersuchungsvorrichtung (4), um die bogenförmige Struktur (2) des vorgefertigten Segments (1) zu formen, und
- Überwachen der von der Verarbeitungseinheit (50) verarbeiteten Daten durch Analyse aller Änderungen in den gesammelten oder verarbeiteten Daten während der Schritte nach dem Betongießen.

12. Produktions- und Überwachungsverfahren (200) nach Anspruch 11, umfassend:

- Vorsehen einer Bewehrung (3) innerhalb der Schalung zum Aufnehmen des Betongusses,
- Befestigen des zumindest einen Untersuchungsgeräts (4) an der Bewehrung (3),
- Wirkverbinden des zumindest einen Untersuchungsgeräts (4) mit einer Verarbeitungseinheit (50), die in der Lage ist, die von zumindest einem Untersuchungsgerät (4) gesammelten Daten zu verarbeiten,
- Durchführen des Betongießens innerhalb der Schalung durch Einbetten der Bewehrung (3) und der zumindest einen Untersuchungsvorrichtung (4), um eine bogenförmige Struktur (2) des vorgefertigten Segments (1) zu formen, und
- Überwachen der von der Verarbeitungseinheit (50) verarbeiteten Daten durch Analysieren aller Spannungsänderungen während der Schritte nach dem Betongießen.

13. Produktions- und Überwachungsverfahren (200) nach Anspruch 11 oder Anspruch 12, umfassend:

- Befestigen des zumindest einen kapazitiven Sensors (10) an einem Träger oder an der Bewehrung (3), bevor das Betongießen ausgeführt wird,
- Wirkverbinden des zumindest einen kapazitiven Sensor (10) mit der Verarbeitungseinheit (50), die in der Lage ist, die von zumindest einem kapazitiven Sensor (10) gesammelten Daten zu verarbeiten, und
- Überwachen der von der Verarbeitungseinheit (50) verarbeiteten Daten durch Analysieren aller strukturellen Veränderungen, die von zumindest einem kapazitiven Sensor (10) während der Schritte nach dem Betongießen erfasst werden.

14. Produktions- und Überwachungsverfahren (200) nach einem der Ansprüche 11 bis 13, umfassend:

- Einschränken des zumindest einen Neigungsmessers (20) auf die Außenseite der bogenförmigen Struktur (2) nach einer vordefinierten Aushärtungszeit (Tc) ab dem Zeitpunkt, an dem das Betongießen stattfand,
- Wirkverbinden des zumindest einen Neigungsmesser (20) mit der Verarbeitungseinheit (50), die in der Lage ist, die von zumindest einem Neigungsmesser (20) gesammelten Daten zu verarbeiten, und
- Überwachen der von der Verarbeitungseinheit (50) verarbeiteten Daten durch Analysieren aller Neigungsänderungen, die von zumindest einem Neigungsmesser (20) während der Schritte nach dem Betongießen erfasst werden.

15. Produktions- und Überwachungsverfahren (200) nach einem der vorhergehenden Ansprüche 11 bis 14, umfassend:

- Warten während der Aushärtezeit (Tc) der bogenförmigen Struktur (2) des vorgefertigten Segments (1),
- Installieren des vorgefertigten Segments (1) innerhalb eines Tunnels, und

- Überwachen der von der Verarbeitungseinheit (50) gesammelten oder verarbeiteten Daten und Analysieren aller erfasster struktureller Veränderungen.

**Revendications**

1. Segment préfabriqué (1) pour un tunnel en béton armé, comprenant une structure arquée (2) ayant une armature (3) et un agglomérat de ciment qui est conçu pour recouvrir des segments de tunnel annulaires structuralement répétés pour des modules correspondant à une fraction de sa section transversale, ladite structure arquée (2) comprenant des faces radiales opposées respectives (2a, 2b) qui sont situées sur des plans qui sont angulairement espacés l'un de l'autre et passant par un axe longitudinal (X) du tunnel, des faces circonférentielles opposées respectives (2c, 2d) qui sont situées sur des surfaces perpendiculaires audit axe longitudinal (X) et sont espacées le long dudit axe longitudinal, des faces longitudinales opposées respectives (2e, 2f) qui sont situées sur des surfaces qui sont parallèles audit axe longitudinal (X), lesdites faces radiales (2a, 2b) étant adaptées pour être déplacées vers des faces radiales respectives de segments adjacents pour former une portion de tunnel annulaire, lesdites faces circonférentielles (2c, 2d) étant adaptées pour être déplacées vers des faces circonférentielles respectives de segments adjacents pour former une étendue linéaire dudit tunnel le long dudit axe longitudinal (X) et une face longitudinale externe (2f), étant à une plus grande distance qu'une face longitudinale interne (2e) dudit axe longitudinal (X), est placée en contact avec le fond dudit tunnel, dans lequel au moins un dispositif d'investigation (4) est noyé dans ladite structure arquée (2) dudit segment préfabriqué (1) à une distance prédéterminée (D) d'au moins l'une desdites faces radiales ( 2a, 2b), circonférentielles (2c, 2d) ou longitudinales (2e, 2f), de manière à détecter des paramètres structuraux prédéterminés, ledit dispositif d'investigation (4) comprend un corps déformable (5) dans lequel est agencé au moins un appareil de mesure de déformation (Ri), **caractérisé en ce que** ledit corps de déformation (5) est configuré pour détecter au moins trois mesures de déformation (E1, E2, E3) orientées l'une par rapport à l'autre, de telle sorte qu'une tension (SYY) au sein dudit dispositif d'investigation (4) est proportionnelle à une combinaison desdites trois mesures de déformation (E1, E2, E3) et **en ce que** ledit corps déformable (5) a un comportement purement élastique, au moins vis-à-vis des contraintes admissibles dans ladite structure dudit agglomérat de ciment.

2. Segment préfabriqué (1) selon la revendication précédente, dans lequel ledit corps déformable (5) comprend deux surfaces (A, B) dont la plus petite dimension est supérieure ou égale au diamètre nominal maximal d'un matériau lié compris dans ledit agglomérat de ciment et a une forme sensiblement aplatie concernant deux dimensions prédominantes de manière à obtenir à l'intérieur dudit corps déformable (5) une zone non perturbée (A2) de ladite tension (SYY), zone dans laquelle ledit au moins un appareil de mesure de déformation (Ri) est agencé.

3. Segment préfabriqué (1) selon l'une quelconque des revendications précédentes, comprenant

   ◦ un premier, un second et un troisième dispositif d'investigation (4a, 4b, 4c) placés respectivement dans lesdites faces radiales opposées (2a, 2b) et dans une zone médiane (M) de ladite structure arquée (2).

4. Segment préfabriqué (1) selon l'une quelconque des revendications précédentes, comprenant

   ◦ au moins un capteur capacitif (10), qui est inclus dans ladite structure arquée (2), pour détecter des fractures internes dudit segment préfabriqué (1).

5. Segment préfabriqué (1) selon la revendication 4, dans lequel

   ◦ ledit au moins un capteur capacitif (10) est logé à proximité d'une zone de butée (S1) identifiée sur une face circonférentielle (2c, 2d) ou sur ladite face longitudinale interne (2e), qui peut être utilisée par un tunnelier pour s'appuyer sur elle pendant une étape de mouvement.

6. Segment préfabriqué (1) selon la revendication 5, dans lequel

   ◦ ledit au moins un capteur capacitif (10) est logé à une distance (F) de ladite face longitudinale interne (2e) qui est égale à approximativement la moitié du pas entre deux zones de positionnement (S1, S2) consécutives de deux vérins de contact dudit tunnelier.

7. Segment préfabriqué (1) selon l'une quelconque des revendications précédentes, comprenant

∘ au moins un inclinomètre (20) agencé dans ou sur ladite structure arquée (2) et conçu pour détecter des variations dans l'ovalisation dudit segment préfabriqué (1).

8. Segment préfabriqué (1) selon la revendication 7, comprenant

∘ une première pluralité d'inclinomètres transversaux (21) qui sont fonctionnellement interconnectés et logés avec un pas prédéfini dans une direction transversale audit axe longitudinal (X), et/ou
∘ une seconde pluralité d'inclinomètres longitudinaux (22) qui sont fonctionnellement interconnectés et logés avec un pas prédéfini dans une direction parallèle audit axe longitudinal (X).

9. Segment préfabriqué (1) selon la revendication 8, dans lequel

∘ ladite première pluralité d'inclinomètres transversaux (21) ou ladite seconde pluralité d'inclinomètres longitudinaux (22) sont positionnés dans ou sur une bande flexible.

10. Anneau de tunnel (100) comprenant

∘ au moins cinq segments préfabriqués, qui sont déplacés les uns vers les autres par deux, sur les faces radiales opposées respectives (2a, 2b) pour fermer ledit anneau,
∘ au moins trois desdits segments préfabriqués étant formés selon les caractéristiques des revendications précédentes.

11. Procédé (200) pour produire et surveiller un segment préfabriqué (1) selon l'une quelconque des revendications précédentes fait en ciment, comprenant

∘ la fourniture d'un coffrage pour recevoir une coulée de béton,
∘ le logement d'au moins un dispositif d'investigation (4) dans ledit coffrage,
∘ la liaison fonctionnelle dudit au moins un dispositif d'investigation (4) à une unité de traitement (50) qui est capable de traiter les données collectées par ledit au moins un dispositif d'investigation (4),
∘ la réalisation de ladite coulée de béton à l'intérieur dudit coffrage en noyant ledit au moins un dispositif d'investigation (4) pour former ladite structure arquée (2) dudit segment préfabriqué (1), et
∘ la surveillance des données traitées par ladite unité de traitement (50) en analysant tout changement dans lesdites données collectées ou traitées pendant les étapes suivant ladite coulée de béton.

12. Procédé de production et de surveillance (200) selon la revendication 11, comprenant

∘ la fourniture d'une armature (3) à l'intérieur d'un coffrage pour recevoir ladite coulée de béton,
∘ la fixation d'au moins un dispositif d'investigation (4) à ladite armature (3),
∘ la liaison fonctionnelle dudit au moins un dispositif d'investigation (4) à une unité de traitement (50) qui est capable de traiter les données collectées par ledit au moins un dispositif d'investigation (4),
∘ la réalisation de ladite coulée de béton à l'intérieur dudit coffrage en noyant ladite armature (3) et ledit au moins un dispositif d'investigation (4) afin de former une structure arquée (2) dudit segment préfabriqué (1), et
∘ la surveillance des données traitées par ladite unité de traitement (50) en analysant tout changement dans la tension pendant les étapes suivant ladite coulée de béton.

13. Procédé de production et de surveillance (200) selon la revendication 11 ou la revendication 12, comprenant

∘ la contrainte d'au moins un capteur capacitif (10) à un support ou à ladite armature (3) avant d'effectuer ladite coulée de béton,
∘ la liaison fonctionnelle dudit au moins un capteur capacitif (10) à ladite unité de traitement (50) capable de traiter les données collectées par ledit au moins un capteur capacitif (10), et
∘ la surveillance des données traitées par ladite unité de traitement (50) en analysant tout changement structural détecté par ledit au moins un capteur capacitif (10) pendant les étapes suivant ladite coulée de béton.

14. Procédé de production et de surveillance (200) selon l'une quelconque des revendications 11 à 13, comprenant

∘ la contrainte d'au moins un inclinomètre (20) sur l'extérieur de ladite structure arquée (2) après un temps de durcissement (Tc) prédéfini à partir du moment où la coulée de béton a eu lieu,

◦ la liaison fonctionnelle dudit au moins un inclinomètre (20) à ladite unité de traitement (50) qui est capable de traiter les données collectées par ledit au moins un inclinomètre (20), et
◦ la surveillance des données traitées par ladite unité de traitement (50) en analysant tout changement d'inclinaison détecté par ledit au moins un inclinomètre (20) pendant les étapes suivant ladite coulée de béton.

**15.** Procédé de production et de surveillance (200) selon l'une quelconque des revendications précédentes 11 à 14, comprenant

◦ l'attente pendant ledit temps de durcissement (Tc) de ladite structure arquée (2) dudit segment préfabriqué (1),
◦ l'installation dudit segment préfabriqué (1) à l'intérieur d'un tunnel, et
◦ la surveillance des données collectées ou des données traitées par ladite unité de traitement (50) en analysant tout changement structural détecté.

*Fig. 1*

Fig. 2

*Fig. 3*

Fig. 4

*Fig. 5*

EP 3 625 437 B1

*Fig. 6*

EP 3 625 437 B1

**EP 3 625 437 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 3033586
- JP 3048027 B
- JP 2011058199 B
- IT 102016000037314